# EUROPEAN PATENT APPLICATION

(11) **EP 3 338 820 A1**
(43) Date of publication of application: **27.06.2018**
(21) Application number: 16205797.0
(22) Date of filing: 21.12.2016
(51) Int. Cl.: A61L 31/10, A61L 31/14, A61L 31/16

(54) **IN SITU PREPARATION OF DRUG ELUTING STENTS WITH BIOCOMPATIBLE PHOTO CROSSLINKED HYDROGELS**

(71) Applicant: Danmarks Tekniske Universitet, 2800 Kgs. Lyngby (DK)
(72) Inventor: MARIZZA, Paolo, 2100 Copenhagen Ø (DK); BOISEN, Anja, 3460 Birkerød (DK)
(74) Representative: Zacco Denmark A/S

(57) **Abstract**

Disclosed herein is a stent system adapted for being implanted in a body cavity or vessel, such as a blood vessel, a ureteral duct or an intestine, the stent system comprising a stent mesh, a catheter extending through the stent mesh, a biocompatible solution adapted for forming photo-crosslinked hydrogels when exposed to radiation, an inner balloon positioned around the catheter, and an outer balloon positioned around the inner balloon, wherein the outer balloon contains the biocompatible solution, the biocompatible solution filling up the space between the inner balloon and the outer balloon.

## Description

The invention relates to a stent system adapted for being implanted in a body cavity or body vessel, such as a blood vessel, an esophageal tract, a biliary or ureteral ducts or an intestine section. The stent system comprises a stent mesh having a hollow cylindrical shape expandable in diameter from an initial stent diameter to an expanded stent diameter, wherein enclosed inside the stent mesh and comprised further in the stent system are a catheter and balloon system containing solution capable of forming a hydrogel.

### BACKGROUND

Stenosis is a stricture, or narrowing of the lumen of a physiologic tubular structure or duct enabling the flow of physiological fluid like blood, urine, bile, food bolus or product of digestion (chime and chyle). The reduction of the lumen diameter may be related to the presence of cholesterol plaques (in blood vessels), bile duct stones, chronic inflammation syndromes (inflammatory bowel disease) and malignant disorders (esophageal,stomach, pancreatic, upper intestine and colorectal cancer).

Strictures in the colon represent a complication of inflammatory bowel disease which interests 1.4 million only in the US. The cause of inflammatory bowel disease is still unknown but is occurs as an abnormal immune response which misidentifies ordinary substances like food, bacteria, and other material trafficking the intestine as harmful pathogens and directs immune cells towards the intestinal epithelium producing local inflammation.

The broadest diseases belonging to inflammatory bowel disease category are Ulcerative Colitis and Crohn's Disease. The prevalence rate of inflammatory bowel disease has been reported to be up to 396 per 100,000 persons. Across the world, the highest incidents occur in the US, Canada, the UK, and Scandinavia.

Colon strictures are recurrent complications for 30% of patients with Crohn's Disease. More than 80% of patients with Crohn's Disease have are submitted to at least one surgical resection in the first 10 years of diagnosis, the operation is due to intestinal strictures. In 40% of the patients, relapse and intestinal strictures need secondary operations. With recurrent resections patients suffer of short bowel syndrome, because intestinal length and function are lost permanently.

The pathophysiology of the inflammatory bowel disease related intestinal stricture has not been clarified yet; it is believed that they result either from persistent inflammation or from fibrosis. When the stenosis is an effect of inflammation the current therapies consist of amino salicylates (sulfazine, mesalamine, olsalazine, balsalazide) and corticosteroid (methylprednisolone, prednisone, budesonide, hydrocortisone) which at present are administered either by the oral or rectal routes. These therapeutics are usefull in the early stage of the disease but they only mitigate the symptoms whilst they do not act on the causes of inflammation.

For patients with advanced disease conditions and for whom the other treatments failed, immunosuppressive and immunoregulatory agents can be considered a therapeutic option. Major drugs in this category are Azathioprine (Imuran®, Azasan®), 6 mercaptopurine (6MP®, Purinethol®), Cyclosporine A (Sandimmune®, Neoral®) and Tracrolimus (Prograf®) available both for oral and parenteral delivery. These active compounds have been used for Crohn's Disease over the last 25 years for decreasing, delaying or preventing strictures. Typically, they exhibit extremely low bioavailability and at the same time can entail harmful over-suppression of immune response increasing the risk of infections and toxicity. Moreover the use of these immunomodulators with such formulations does not change risk of developing intestinal strictures. A clever drug delivery system is here needed to optimize the drug concentration at the site of action and enhance the drug bioavailability by enhancing drug aqueous solubility and promoting drug absorption by means of adequate pharmacological carriers.

The newest class of biological therapeutics, approved by FDA for treatment of inflammatory bowel diseases include antibodies and peptides targeting the specific substances involved in the overactive immune response. To this group belong Infliximab (Remicade®, Adalimumab (Humira®), Certolizumabpegol (Cimzia®), Natalizumab (Tysabri®), Golimumab (Simponi®). Humira and Remicade were reported to be amongst the top 15 bestselling drugs of 2012. So far all the biologics are available for parenteral administration only because there are no available formulations which can preserve the therapeutic activity of these labile compounds from the aggressive environment of the digestive system.

For orally dosed therapeutics the availability to the diseased sites exhibits large inter-individual differences (between 35 and 75%) resulting in variable clinical response rates mainly for factor connected to intestinal physiology of the disease: (i) prolonged intestinal transit time (compared to healthy patients), (ii) intraluminal pH profiles, (iii) variable distribution pattern of inflamed mucosa. Indeed an effective therapeutic action can be achieved only with defined drug concentrations in the mucosa, considering that degradation and deactivation by the intestinal microflora as well as systemic circulation of the drug should be limited to reduce the potential for adverse effects. On the other hand, the major disadvantage of the biologics administered by injections is that they lack of any targeting ability meaning that by the systemic circulation they travel throughout the body suppressing the immune system. In the ideal case the active ingredient should be released in proximity to the affected areas and execute topically its therapeutic action.

Along with the pharmacological therapies, Crohn's Disease related anastomotic strictures up to 4-5 cm long have been treated by endoscopic balloon dilation with long term success the in 41%-63% of patients since 1970s, but the remaining group of patients still requires repeated dilation and surgery.

To reduce this high risk of recurrence, the use of stents had been proposed. A stent is a cylindrical medical device with a reticular structure with expandable properties which is inserted in the body cavity to widen a narrow or stenosed lumen and maintain its physiological shape and dimension. Currently, stents are being increasingly used in blood vessels, esophagus, renal and biliary ducts and in the gastrointestinal and colonic tracts.

Over the last 30 years, material and design of stents with gastrointestinal applications, referred as enteral stents, have undergone dramatic changes. Materials proposed for these stents were initially plastic, then metallic alloys until the last generation of biodegradable stents which is currently under clinical studies. Enteral stents are meant to produce endoscopic stricturoplasty with a prolonged dilation of the stenosis compared to the endoscopic balloon. The main drawbacks of these devices which so far limited their clinical use are the possible stent migration and perforation of the surrounding mucosa. Covered stents have been introduced to minimize tissue ingrowth through the mesh but are associated with higher occurrence of migration.

The geometry of the stent coating has a strong impact on stent spontaneous movements, and a control of the coating is very important feature. Another possible function of the coating is the local release of active ingredient towards the surrounding intestinal tract and in the whole colonic region. Drug eluting stents (DES) have been initially introduced in angioplasty procedure to reduce proliferation of smooth muscle cells and vessel wall recoil following the insertion of stents in region obstructed by cholesterol plaques. As for other implantation sites, drugeluting enteral stents are likely becoming available in the near future. Experts in the field agreed to identify the most important features for DES in the following: (i) homogeneous coverage of the drug along all stent surfaces; (ii) retention and protection of the drug on the stent when exposed to routine handling; (iii) prevention of the loss of drug during processing and stent implantation; (iv) prevention of overdosing from non-uniform drug distribution or immediate burst release of the total drug payload; (v) local, targeted, short-term, low-dose drug release.

Drug eluting stents currently under study are loaded with small molecule chemotherapeutic and antiproliferative drugs by pre-insertion deposition by micro pipetting and drying. This procedure requires particular ability in the specialized personnel in the execution of stent positioning, as a critical aspect is represented by loss of substance during the handling. Moreover, prior to administrating novel active ingredients, like the abovementioned peptide and antibody based- compounds (and the eventual carrier in which they are encapsulated), have to be maintained in an aqueous environment with defined conditions of pH, temperature and ionic strength, in order to maintain their therapeutic activity. The stent deposition procedures so far explored are unlikely applicable for such labile therapeutics. The coating distribution on the stent structure is also an important issue: the drug elution should preferably be oriented towards the cavity walls and release the therapeutic agent unidirectionally while the release to the lumen side should be avoided to reduce drug loss and unspecific adsorption by healthy tissues. To fulfill a unidirectional drug elution stents with special nanoliter microreservoir open on the external surface were proposed. Besides the high costs such devices suffer of low capacity for the drug payload and rather complicated loading procedure are required.

As a result, an *in situ* deposition of the liquid coating after the stent positioning would allow to extend optimal physico-chemical conditions for labile formulations until prior to administration, and high control of the drug dose for the minimal handling is required. Such a coating would maintain intact the therapeutic efficacy of the most novel formulations (promoting internalization of the therapeutic in the diseased cells) and would be anchored to the stent mesh in such a way that reduces stent migration and limits the drug release towards the lumen side.

When local occlusions occurs in a body cavity or body vessel such as e.g. a blood vessel, an esophageal tract, a ureteral duct or an intestine section, biocompatible stents are often inserted into the body cavity/vessel as treatment.

A stent with a small cross-section may be inserted into the body cavity/vessel/lumen by means of a catheter and expended at the correct location inside the body cavity/vessel by use of an expanding balloon positioned inside the mesh.

Alternatively, a stent may be formed *in situ* as shown in EP0836448B1, which discloses a curable fibre composite stent, which, upon formation *in situ* inside a selected region of a body lumen, serves to support the lumen without significant obstruction to blood flow. The stent is made up of a biocompatible fibrous material which is coated, impregnated, filled, or otherwise treated with a curable material so that the fibre composite can be suitably shaped to support a portion of a body lumen and then cured to maintain the shape. The fibre composite and the curable material may be biodegradable so that the stent does not become a permanent part of the body. The polymer stent may be cured using UV light which is inserted through the catheter/balloon system in situ.

Alternatively, a stent may be formed inside a body cavity as disclosed in WO2011/130536A2, where liquid biodegradable material are formed as stents inside the body by means of a catheter, a balloon system comprising three balloons, and UV light. In WO2011/130536A2, two balloons are expanded whereby a cavity is formed into which a polymer material is introduced. A third balloon is used for obtaining a specific shaping of the polymer material, and UV light is used afterwards for illuminating the polymer solution to cure the polymer. The polymer stents formed in WO2011/130536A2 are biodegradable and will therefore degrade over time.

EP0431046B1 also discloses a polymer stent fabricated and tested *in vivo* by placing polymer material on a balloon, which is inserted and shaped inside the human body cavity. Afterwards the polymer is illuminated with UV light to obtain a more rigid solid structure by curing the polymer material. The stent is made from a biodegradable polymeric materials such as e.g. a polylactone or a polyanhydride in a form of solid matrix capable of maintaining the size and shape and proving mechanical support to the tissue around it.

### DESCRIPTION OF THE INVENTION

The invention relates in a first aspect of the invention to a stent system adapted for being implanted in a body cavity or vessel. Examples of body cavities/vessels are a blood vessel, a ureteral duct, an esophageal tract, an intestine section, or similar. Thus, by body cavity or body vessel is meant all types of channels or lumens inside the human body ranging from small blood vessels to large intestines.

The stent system comprising a stent mesh with a stent structure, the stent mesh having a hollow cylindrical shape expandable in diameter from an initial stent diameter to an expanded stent diameter, the hollow cylindrical shape comprising a plurality of openings in between the mesh structure. The stent mesh may also be expandable or shrinkable in a longitudinal direction.

Enclosed inside the stent mesh and comprised further in the stent system are:
- a catheter extending through the stent mesh, the catheter being adapted for containing an optical fibre providing radiation, e.g. in the wavelength range between 10-800 nm;
- a biocompatible solution adapted for forming photo-crosslinked hydrogels when exposed to radiation from the optical fibre extending through the catheter, the biocompatible solution comprising one or more monomers, and/or one or more oligomers, and/or one or more polymers;
- an inner balloon positioned around the catheter;
- an outer balloon positioned around the inner balloon, wherein the outer balloon contains the biocompatible solution, the biocompatible solution filling up the space between the inner balloon and the outer balloon.

Upon expansion of the inner balloon, the stent mesh may expand to obtain the expanded stent diameter, and the outer balloon breaks whereby the biocompatible solution is distributed in the openings in between the stent mesh structure and around the stent mesh structure. By around is also meant the space between the stent mesh and the body cavity/vessel.

By photo-crosslinked is also included photo-reticulation - i.e. any linking reaction between the biocompatible solution comprising one or more monomers, and/or one or more oligomers, and/or one or more polymers.

Alternatively, the stent mesh may be self-expandable, possibly expanding prior to the expansion of the balloons.

Upon radiation from the optical fibre, the biocompatible solution distributed in the openings in between the stent mesh structure and around the stent mesh structure is adapted to create a coating layer around the stent mesh.

The stent system will most often be implanted in an inflamed section of the body cavity/vessel, or a section, which needs further support in order to avoid collapse of the cavity/vessel.

The invention relates in a second aspect of the invention to a method for implanting a hydrogel coated stent in a body cavity or body vessel, such as a blood vessel, a ureteral duct or an intestine section. The method comprising the steps of:
- inserting a stent system according to the above into a body cavity or body vessel;
- inserting an optical fibre adapted for providing light in the wavelength range between 10 nm - 800 nm inside the stent system if the optical fibre is not already inserted inside the stent system;
- expanding the inner balloon to a pre-determined size such that the stent mesh obtains a first predetermined size at which the outer balloon breaks and the biocompatible solution is distributed in the openings in between the stent mesh structure and around the stent mesh structure;
- shaping the stent mesh to match the tissue in the surrounding body cavity or body vessel by expanding the inner balloon to obtain the expanded diameter;
- irradiating the biocompatible solution distributed in the openings in between the stent mesh structure and around the stent mesh structure with light in the wavelength range between 10 nm - 800 nm from the optical fibre thereby creating a reticulated polymeric hydrogel serving as a coating of the stent mesh, and
- removing the optical fibre and catheter from the body cavity or body vessel thereby leaving the hydrogel coated stent in the body cavity or body vessel.

The invention relates in a third aspect of the invention to a method for implanting a hydrogel coated stent in a body cavity or body vessel, such as a blood vessel, a ureteral duct or an intestine, the method comprising the steps of:
- inserting a stent system according to the above into a body cavity or body vessel;
- inserting an optical fibre adapted for providing light in the wavelength range between 10 nm - 800 nm inside the stent system if the optical fibre is not already inserted inside the stent system;
- expanding the stent mesh to match the body cavity or body vessel;
- expanding the inner balloon to a pre-determined at which the outer balloon breaks and the biocompatible solution is distributed in the openings in between the stent mesh structure and around the stent mesh structure;
- possibly shaping the stent mesh to match the tissue in the surrounding body cavity or body vessel by expanding the inner balloon to obtain the expanded diameter;
- irradiating the biocompatible solution distributed in the openings in between the stent mesh structure and around the stent mesh structure with light in the wavelength range between 10 nm - 800 nm from the optical fibre thereby creating a reticulated polymeric hydrogel serving as a coating of the stent mesh, and
- removing the optical fibre and catheter from the body cavity or body vessel thereby leaving the hydrogel coated stent in the body cavity or body vessel.

By body cavity or body vessel is meant all types of channels or lumens inside the human body ranging from small blood vessels to large intestines.

By the biocompatible solution being distributed in the openings in between the stent mesh structure and around the stent mesh structure is meant that the solution surrounds the stent mesh structure on the inside and the outside as well in the openings between the mesh structure.

The optical fibre may provide radiation in the ultra violet wavelength range or at both higher or lower wavelength ranges, e.g. between 10-800 nm. For example, the optical fibre may provide radiation in the ultra violet wavelength range between 10-450 nm.

In one or more embodiments, the optical fibre may provide radiation in the wavelength range between 100-450 nm, or between 150-400 nm, or between 300-400 nm.

Alternatively, the optical fibre may provide radiation in the visible wavelength range between 400-800 nm. The wavelength range extending a bit into the near infrared and the ultra violet wavelength range.

In one or more embodiments, the optical fibre may provide radiation in the wavelength range between 450-700 nm, or between 400-500 nm, or between 500-600 nm, or between 600-700 nm.

In yet alternative embodiments, the optical fibre may provide radiation in the infrared wavelength range above 700 nm, e.g. between 700-2000 nm. In one or more embodiments, the optical fibre may provide radiation in the wavelength range between 700-800 nm, or between 800-1200 nm, or between 1100-2000 nm.

The choice of wavelength is made to ensure that a cross-linked hydrogel coating is obtained as well as to ensure that as little damage to the surrounding tissue/vessel wall is introduced due to radiation from the radiation source *in situ.*

By the above stent system and method is obtained a system/method, which allows a practitioner to insert a biocompatible stent inside a body cavity/vessel of a patient and *in situ* form a coating layer around the stent mesh. This coating layer has a number of highly beneficial properties, one of them being that it is a hydrogel coating layer having a biosimilar nature, thus being biocompatible. The biocompatibility ensures an improved adhesion between the stent mesh and the surrounding tissue/vessel wall in the body cavity/vessel. Furthermore, the hydrogel coating layer reduces the occurrence of spontaneous movement of stent after insertion in the body cavity/vessel, which is a problem very frequently observed with e.g. well-known enteral stents. Also, the hydrogel coating layer around the stent mesh prevents tissue perforation by sharp fibres in the stent mesh. The hydrogel coating layer further offers a mechanical resistance to reocclusion of the lumen caused by cell regrowth and/or fibrotic tissue growth and/or immunogenic reactions. Also, the hydrogel coating reduces the occurrence of restenosis. Further, the hydrogel coating is suitable for drug/therapheutic substance delivery purposes. For example, is it possible to incorporate liposomes and other colloidal formulations in their functional state.

In one or more embodiments, the stent mesh is adapted for expanding to obtain the expanded stent diameter upon expansion of the inner balloon. Alternatively, the stent mesh may have self-expanding features.

Depending on the size of the body cavity/vessel, which the stent system is to be inserted in, the size of the stent mesh and the coating layer formed around the mesh will vary. Overall, the stent mesh may have an initial stent mesh diameter of between 2-30 mm. Similarly, overall the coating layer may have a thickness between 0.05 µm - 5 mm,
If the stent system is for being inserted into a blood vessel, the stent mesh is normally rather small with an initial non-expanded stent diameter of 2-5 mm, which is expanded *in situ* to fit inside the blood vessel. Thus, in one or more embodiments, the stent mesh has an initial stent diameter of 2-5 mm.

In one or more embodiments, the coating layer has a thickness between 0.1 µm - 10 µm.

In one or more embodiments, the coating layer has a thickness between 0.1 µm - 1 µm.

In one or more embodiments, the coating layer has a thickness between 1 µm - 100 µm.

In one or more embodiments, the coating layer has a thickness between 1 µm - 10 µm.

If the stent system is for being inserted into a larger body channel such as e.g. a ureteral duct, the stent mesh will normally need to be larger than the stent meshes used for a blood vessel. Such a stent mesh may have an initial non-expanded stent diameter of 8-10 mm, which is expanded *in situ* up to size matching the inside the ureteral duct. Thus, in one or more embodiments, the stent mesh has an initial stent diameter of 8-10 mm.

In one or more embodiments, the coating layer has a thickness between 10 µm - 100 µm.

In one or more embodiments the coating layer has a thickness between 10 µm - 500 µm.

In one or more embodiments the coating layer has a thickness between 1 µm - 1 mm.

In one or more embodiments the coating layer has a thickness between 1 mm - 5 mm.

The coating layer thicknesses between 1-5 mm are usually more suitable for large stent mesh sizes such as those used in the intestine part. The stent mesh for use in the intestine will normally have an initial non-expanded stent diameter of 2-30 mm, which is expanded *in situ* up to match the inside of the intestine. Thus, in one or more embodiments, the stent mesh has an initial stent diameter of 2-30 mm.

The biocompatible solution comprises one or more monomers, and/or one or more oligomers, and/or one or more polymers. Examples of suitable polymers are polyvinylpyrrolidone, sodium alginate, synthetic polymers, or naturally occurring polymers modified to be cross-linkable upon exposure to radiation from the optical fibre.

In one or more embodiments, the one or more polymers is polyvinylpyrrolidone.The hydrogel with polyvinylpyrrolidone used in an example of the invention have proven to have antifouling properties.

In one or more embodiments, the biocompatible solution is a hydrogel solution having a three dimensional crosslinked polymer network with amphiphilic components.

In one or more embodiments, the biocompatible solution further comprises one or more additional substances. The additional substance may -when exposed to radiation - serve the purpose of photo initiating/activating the hydrogel polymerization and/or cross-linking reaction to form the hydrogel coating layer. Thus, the stent system may further comprise a photo-initiator/cross-linker in connection with/contained inside the biocompatible solution. The hydrogel coating layer may in this manner be formed by a radical or ionic cross-linking or polymerization reaction initiated by the photo initiator when the photo initiator is exposed to radiation from the optical fibre.

In one or more embodiments, the biocompatible solution comprises two or more polymers, wherein at least one of the polymers is cross-linkable upon exposure to radiation between 10 nm - 800 nm. The cross-likable polymer may serve as photo initiator.

In one or more embodiments, the photo initiator is a water soluble photo initiator.

In one or more embodiments, the additional substance is in a concentration between 0.1-1.5% wt., or between 0.5-1.5% wt., or between 0.7-1.0% wt., such as e.g. 0.9% wt.

In one or more embodiments, the one or more additional substances is selected from the group of: hydrogen peroxide, benzoyl peroxide, acetophenone, benzoin, 2,2-dimethoxy-2-phenylacetophenone, dibenzoyl, disulphides, diphenyldithiocarbonate, 2,20-azobisisobutyronitrile, camphorquinone, eosin Y, triethanolamine, 2,2-bis[4-(2-hydroxy-3-methacryloxypropoxy)-phenyl]propane, lithium phenyl-2,4,6-trimethylbenzoylphosphinate, (1-[4-(2-Hydroxyethoxy)-phenyl]-2-hydroxy-2-methyl-1-propane-1-one, 2,20-Azobis[2-methyl-N-(2-hydroxyethyl)propionamide, (1-hydroxycyclohexyl)-phenylmethanone.

The additional substance may be hydrogen peroxide which in small concentrations only has an ignorable toxic effect on the tissue in the body cavity/vessel.

In one or more embodiments, one or more bioactive components are comprised in the stent system.

The one or more bioactive components may include one or more of: a bioactive agent, a bioactive principle, a bioactive compound, a bioactive molecule, a bioactive drug, a therapeutic agent, a therapeutic principle, a therapeutic compound, a therapeutic molecule, and a therapeutic drug.

In one or more embodiments, the one or more bioactive components is a bioactive component for preventing that the immune response in the body is activated. If this happens, in worse case scenario, the body might try to reject the stent mesh.

In one or more embodiments, the one or more bioactive components is a bioactive component for preventing that the tissue in the body cavity/vessel is injured due to the stent mesh.

In one or more embodiments, the one or more bioactive components is a bioactive component for preventing inflammation or similar inside the body.

In one or more embodiments, the one or more bioactive components is a bioactive component for postponing that the immune response in the body is activated. If this happens, in worse case scenario, the body might try to reject the stent mesh.

In one or more embodiments, the one or more bioactive components is a bioactive component for postponing inflammation or similar inside the body.

In one or more embodiments, the one or more bioactive components is a therapeutically active component for postponing a disease, and inflammation or similar. inside the body.

In one or more embodiments, the one or more bioactive components is a therapeutically active component for treating a disease, and inflammation or similar inside the body.

This allows for using the stent system for delivering both 1) a bioactive component, which prevents rejection of the stent or inflammation, which may result in the formation of scar tissue around the stent, and 2) at the same time delivering a drug or similar, which reduces/eliminates inflammation or cures a disease in or around the body cavity/vessel, which the stent system is inserted into.

In one or more embodiments, a first and a second bioactive component is included in the stent system such that at least a prevention of the stent being rejected is obtained at the same time as a drug for therapeutic purposes is delivered to the tissue around the stent.

In one or more embodiments, the one or more bioactive components is a synthetic or a natural compound of a molecular weight ranging from 100 to 500.000 Da, such as from 1.000 to 500.000 Da, or such as from 10.000 to 500.000 Da, or such as from 100 to 10.000 Da, or such as from 100 to 1.000 Da.

The smaller bioactive components having an average weight ranging from 100 to 1.000 Da exhibit also poor solubility in water, and often have issues with permeability in the biological tissue. For such components it is difficult to reach the target tissue with a sufficient therapeutic concentration without the use of a stent system according to the invention.

The larger molecules, like macromolecules having an average weight ranging from 1000 to 500.000 Da, are normally unstable due to a more fragile structure. Further, the large macromolecules normally have permeability issues and are unlikely to get absorbed without a system that forces them to stay in proximity to the target tissue such as the coating described in this invention. Specific delivery and distribution of macromolecules to an exact location in the body before they start to degrade, are methabolized or cleared or lose their bio-relevant activity are normally very difficult if not impossible without the use of a stent system according to the invention.

To enhance the availability of fragile macromolecules the site of release should be in close proximity to the target tissue as it is obtained from the hydrogel coating in this invention. Both the dispersion of small hydrophobic therapeutic compounds in aqueous environment and of the internalization of the therapeutic macromolecule in the targeted cells is provided by a clever drug delivery system further to be comprised in the biocompatible solution forming the stent coating upon photo-reticulation / photo-crosslinking.

Thus, the stent system disclosed herein can be advantageously used for delivering bioactive components of all kind of sizes.

The one or more bioactive components may in one or more embodiments be contained inside colloidal formulations. An example of a colloidal formulations includes colloidal drug delivery system (DDS) such as liposomes. Liposomes are a specific colloidal drug delivery system (DDS) where the drug is complexed or encapsulated inside a spherical lipid nano vesicular carrier suspended in an aqueous phase. In such a DDS, the drug compound (may it be hydrophobic, hydrophilic, a small and big molecule) is protected from the liquid reagents constituting the hydrogel solution. How fast the DDS is released form the hydrogel coating to the tissue in the body cavity/vessel depends on the DDS used and/or on the hydrogel coating structural properties. In this manner, controlled release of different drug components can be obtained by using different DDS and or hydrogel coatings. Thus, the therapeutic formulation may be released by the hydrogel coating over time in a controlled manner. The drug is normally not affected or deactivated by the radiation from the optical fibre due to the use of the nano vesicular carrier. The nano vesicular carrier may be spherical and formed by lipids and surfactants.

The degree of crosslinking of the hydrogel coating may be controlled by the light intensity/irradiation time or by adding different photo initiators and/or photo crosslinking agents and different polymers to the solution and/or changing their respective concentrations. In turn this allows for a control of the release of the bioactive compounds towards the body cavity/vessel. The release may be controlled such that an amount of the bioactive agent is released towards the centre of the body cavity/vessel from where the bioactive agent, e.g. a drug, may be flushed by flow of the body fluid stream, e.g. the blood stream. Alternatively, the release may be controlled such that it is primarily directed to the cavity/vessel walls of the body cavity/vessel, whereby only a very small amount is flushed by flow of the body fluid stream, e.g. the blood stream. Alternatively, the release may be controlled such that the entire amount of drug payload stored inside the hydrogel coating is released to the target tissue within the needed therapeutic ranges of concentration and time.

For example, a therapeutic formulation may upon radiation be fixed inside an aqueous medium retained in a hydrogel three dimensional crosslinked polymer network serving as the stent coating. This ensures intact and functional properties for the delivery purpose of the therapeutic agent.

In one or more embodiments, one or more drug components in the DDS is a hydrophobic drug molecule.

In one or more embodiments, one or more drug components in the DDS is a hydrophilic drug molecule.

In one or more embodiments, the one or more bioactive components, possibly comprised inside the colloidal formulation, are comprised in the biocompatible solution.

Alternatively or supplementary, in one or more embodiments, the one or more bioactive components, possibly comprised inside the colloidal formulation, are contained inside the micro reservoirs in the stent mesh. The stent mesh may be micro fabricated.

The hydrogel coating may have a three dimensional crosslinked polymer network with amphiphilic components having entrapped in its three dimensional structure a colloidal formulation containing bioactive agents comprising synthetic or natural polymers which are proteins or mixtures thereof.

The stent mesh may be made from a biodegradable material. This is advantageous if the stent is meant to dissolve inside the patient over time.

Alternatively, the stent mesh is made from biocompatible and/or biodegradable metal. This is very useful if it is critical that the body cavity/vessel remains dilated, as a metal mesh will retain its structure and not collapse over time. Examples of suitable biocompatible metals includes titanium alloys, zinc or zinc alloys, cobalt-chromium alloys, magnesium or magnesium alloys, and stainless steel.

### BRIEF DESCRIPTION OF THE DRAWINGS

Figures 1 a-b show a stent system according to the invention.
Figures 2a-f show the implantation of a stent system according to the invention.
Figure 3 shows the concentration of liposomes labelled with fluorescein which are released over time by different hydrogels specimens composed by a single polymer constituent or by a blend of two polymer constituents in different concentrations.
Figure 4 shows the nano scale structure of a hydrogel network observed with transmission electron microscopy.

### DESCRIPTION OF PREFERRED EMBODIMENTS

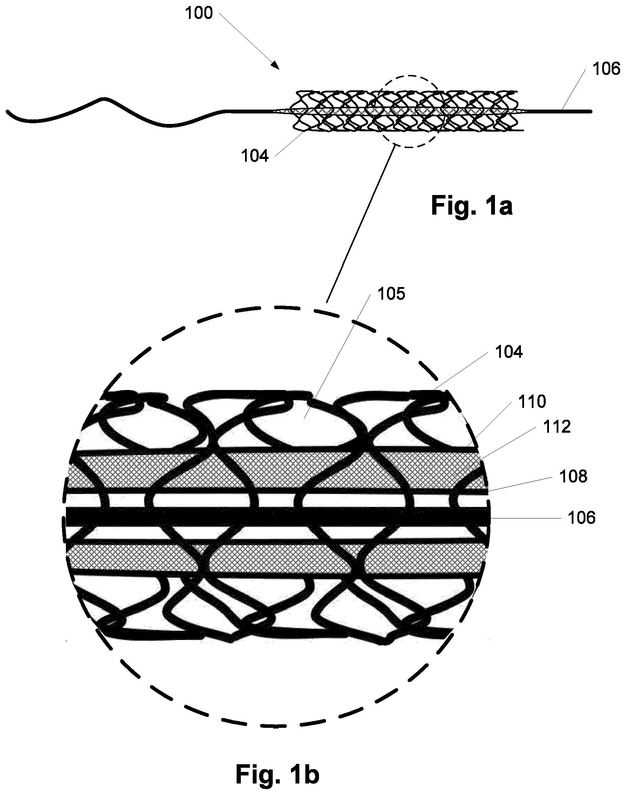

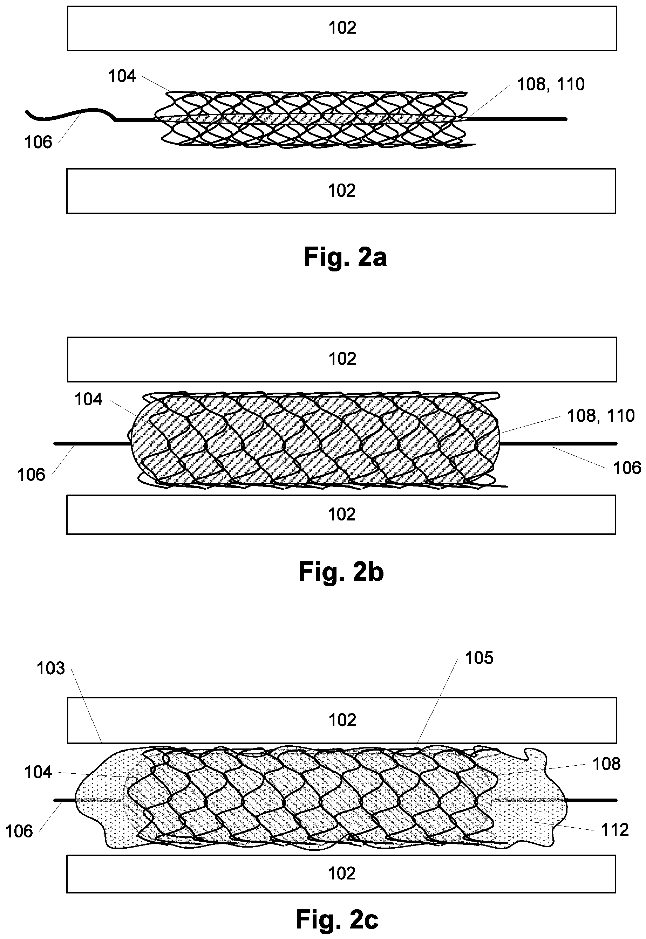

Figure 1 shows an example of the invention, where the circle marked in figure 1 a is the enlargement shown in figure 1b. In figure 1a, a stent system 100 adapted for being implanted in a body cavity/vessel 102 as shown figures 2a-e is shown. The stent system 100 will most often be implanted in an stenosed/inflamed section of the body cavity/vessel 102 to restore its physiological diameter section, which needs further support in order to avoid recollapse of the cavity/vessel after implantation.

The stent system 100 comprises a stent mesh 104 having a hollow cylindrical shape expandable in diameter from an initial stent diameter to an expanded stent diameter. In figures 2a-e, the expansion and the implantation and *in situ* coating of the stent 104 are illustrated. As can be seen in figure 1b, the hollow cylindrical shape of the stent mesh comprising a plurality of openings 105 in between the mesh structure.

As also shown in figure 1b, enclosed inside the stent mesh 104 and comprised further in the stent system 100 is a catheter 106 extending through the stent mesh 104. The catheter 106 has an inner diameter which is large enough for allowing an optical fibre to extend there through. The optical fibre may be included in the catheter 106 upon delivery of the stent system 100 or may be a separate fibre, which the medical practitioner inserts through the catheter 106 prior to insertion of the stent system 100 in a human body cavity/vessel. Thus, in the figures the reference number 106 refers both to a catheter without/before an optical fibre extends there through and a catheter containing an optical fibre. The optical fibre may provide radiation in the ultra violet wavelength range or at both higher or lower wavelength ranges, e.g. between 10-800 nm.

Surrounding the catheter 106 is a balloon system including an inner balloon 108 positioned directly around the catheter 106 and an outer balloon 110 positioned around the inner balloon 108. In between the two balloons is a space filled with a biocompatible solution 112, which can form reticulated hydrogels, e.g. photo-crosslinked hydrogels, when exposed to radiation from the optical fibre extending through catheter 106.

The biocompatible solution 112 comprising one or more monomers, and/or one or more oligomers, and/or one or more polymers.

Upon expansion of the inner balloon 108 - and thereby also the outer balloon 110 - the stent mesh 104 expands to obtain the expanded stent diameter. This results in the outer balloon 110 breaking whereby the biocompatible solution 112 is distributed in the openings 105 in between the stent mesh structure and around the stent mesh.

Alternatively, the stent mesh 104 may have self-expanding features such that the expansion of the stent mesh 104 is not directly obtained by the expansion of the balloons 108, 110. In that situation, the expansion will most often occur before the expansion if the inner balloon.

Upon radiation from the optical fibre, the biocompatible solution 112 distributed in the openings in between the stent mesh structure and around the stent mesh structure is adapted to create a hydrogel coating layer 114 around the stent mesh structure (see figure 2c).

Depending on the size of the body cavity/vessel, which the stent system 100 is to be inserted in, the size of the cylindrical mesh 104 and the coating layer 114 formed around the mesh 104 varies.

If the stent system 100 is for being inserted into a blood vessel, the stent is normally rather small with expanded diameter fitting inside the blood vessel. If the body cavity/vessel instead is an intestine, it may have a large expanded diameter and stents for body cavities of sizes in between that of a blood vessel and an intestine, will expand to a middle sized expanded stent diameter having the size of e.g. an ureteral duct.

Figures 2a-e show how a hydrogel coated stent implanted in a body cavity/vessel 102, such as a blood vessel, a ureteral duct or an intestine starting with the stent system disclosed herein.

Figure 2a shows a stent system inserted in a body cavity/vessel 102. As can be seen in the figure, the stent mesh 104 is in the initial configuration, where the diameter of the stent mesh is small. Also none of the balloons 108, 110 are expanded yet. Through the catheter 106 extends an optical fibre (not visible form the figure) adapted for providing light between 10 nm - 800 nm. Normally, ultra violet, visible, or infrared light will be provided from the fibre.

Figure 2b shows the stent system after the inner balloon 108 - and thereby also the outer balloon 110 - has been expanded nearly to a first pre-determined size at which the outer balloon 110 breaks and the biocompatible solution 112 is distributed in the openings 105 in between the stent mesh structure and around the stent mesh structure as shown in figure 2c

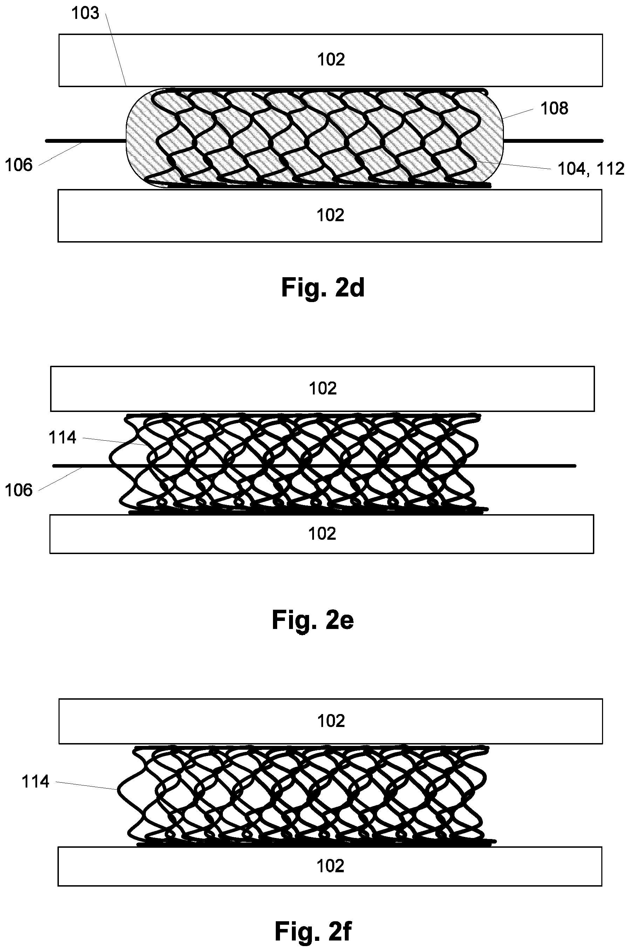

Figure 2c shows the stent system after the outer balloon 110 has broken and the biocompatible solution 112 is squeezed against/around and in between the stent mesh structure also filling some of the space between the stent mesh 104 and the body cavity/vessel 102. The shaping of the stent mesh 104 to match the tissue/vessel wall 103 in the surrounding body cavity/vessel 102 by expanding the inner balloon 108 further to obtain an expanded diameter is illustrated in figure 2d. In figure 2d, the lines symbolising the stent mesh 104 have been drawn thicker to illustrate that the biocompatible solution 112 is now surrounding the stent mesh structure 104.

Figure 2e illustrates the final irradiation of the biocompatible solution 112 distributed in the openings in between the stent mesh structure and around the stent mesh structure 104 with light between 10 nm - 800 nm from the optical fibre extending through the catheter 106. The irradiation process starts a polymerization and/or cross-linking reaction of the biocompatible solution 112 which creates a hydrogel coating 114 surrounding the stent mesh structure 104.

After the hydrogel coating 114 has been formed, the optical fibre and the catheter 106 are removed from the body cavity/vessel 102 thereby leaving the hydrogel coated stent in the body cavity/vessel as shown in figure 2f.

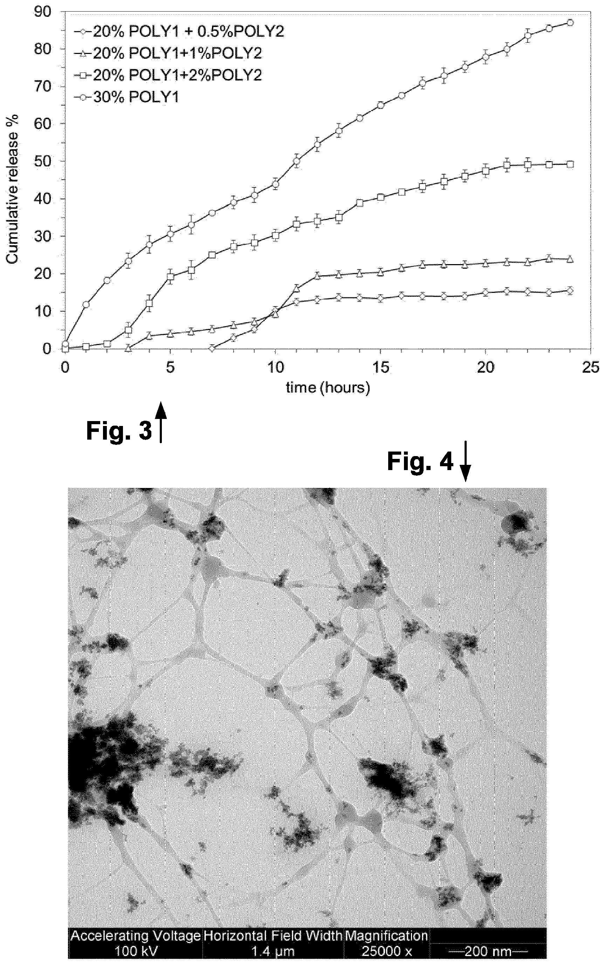

Figure 3 shows the concentration of liposomes labelled with fluorescein which are released over time by different hydrogels specimens composed by a single polymer constituent or by a blend of two polymer constituents in different concentrations. In the specific example, the abbreviation POLY1 refers to polyvinylpyrrolidone (PVP) and the abbreviation POLY2 refers to sodium alginate.

The different kinetic trends displayed in figure 3 show the cumulative release as a function of time. Depending on the therapeutic necessity, the release rate may be tuned and controlled by variations of the monomer/oligomer/polymer type/mixtures in the biocompatible solution.

Figure 4 shows the nano scale structure of the polyvinylpyrrolidone and sodium alginate mixture hydrogel network measured using Cryogenic Transmission Electron Microscopy.

### REFERENCES

- 100: stent system
- 102: body cavity/vessel
- 103: wall of the body cavity/vessel
- 104: stent mesh
- 105: openings in the stent mesh
- 106: catheter
- 108: inner balloon
- 110: outer balloon
- 112: biocompatible solution
- 114: hydrogel coating layer around/surrounding the stent mesh structure

## Claims

1. A stent system adapted for being implanted in a body cavity or body vessel, such as a blood vessel, a ureteral duct or an intestine section, the stent system comprising a stent mesh with a stent structure, the stent mesh having a hollow cylindrical shape expandable in diameter from an initial stent diameter to an expanded stent diameter, the hollow cylindrical shape comprising a plurality of openings in between the mesh structure, wherein enclosed inside the stent mesh and comprised further in the stent system are:
- a catheter extending through the stent mesh, the catheter being adapted for containing an optical fibre providing radiation, e.g. in the wavelength range between 10-800 nm;
- a biocompatible solution adapted for forming photo-crosslinked hydrogels when exposed to radiation from the optical fibre extending through the catheter, the biocompatible solution comprising one or more monomers, and/or one or more oligomers, and/or one or more polymers;
- an inner balloon positioned around the catheter;
- an outer balloon positioned around the inner balloon, wherein the outer balloon contains the biocompatible solution, the biocompatible solution filling up the space between the inner balloon and the outer balloon,
wherein upon expansion of the inner balloon, the stent mesh expands to obtain the expanded stent diameter, and the outer balloon breaks whereby the biocompatible solution is distributed in the openings in between the stent mesh structure and around the stent mesh structure,
wherein the biocompatible solution distributed in the openings in between the stent mesh structure and around the stent mesh structure upon radiation from the optical fibre is adapted to create a coating layer around the stent mesh.

2. A stent system according to claim 1, wherein the coating layer has a thickness between 0.05 µm - 5 mm, such as between 0.1 µm - 1 mm, such as between 0.1 µm - 100 µm, such as between 0.1 µm - 10 µm, such as between 0.1 µm - 1 µm, such as between 1 µm - 100 µm, such as between 1 µm - 10 µm, such as between 1 mm - 5 mm, such as between 10 µm - 100 µm, such as between 10 µm - 500 µm, such as between 1 µm - 1 mm.

3. A stent system according to any preceding claim, wherein the biocompatible solution comprises two or more polymers, wherein at least one of the polymers is cross-linkable upon exposure to radiation between 10 nm - 800 nm.

4. A stent system according to any preceding claim, wherein the biocompatible solution is polyvinylpyrrolidone, sodium alginate, synthetic polymers, or naturally occurring polymers modified to be cross-linkable upon exposure to radiation from the optical fibre.

5. A stent system according to any preceding claim, wherein the biocompatible solution further comprises one or more of the following additional substances:
• hydrogen peroxide
• benzoyl peroxide
• acetophenone
• benzoin
• 2,2-dimethoxy-2-phenylacetophenone
• dibenzoyl disulphides
• diphenyldithiocarbonate
• 2,20-azobisisobutyronitrile
• camphorquinone
• eosin Y
• triethanolamine
• 2,2-bis[4-(2-hydroxy-3-methacryloxypropoxy)-phenyl]propane
• lithium phenyl-2,4,6-trimethylbenzoylphosphinate
• (1-[4-(2-Hydroxyethoxy)-phenyl]-2-hydroxy-2-methyl-1-propane-1-one
• 2,20-Azobis[2-methyl-N-(2-hydroxyethyl)propionamide
• (1-hydroxycyclohexyl)-phenylmethanone.

6. A stent system according to any preceding claim, wherein the stent system further comprises a photo initiator/cross-linker in connection with the biocompatible solution, and wherein the coating layer is formed by a radical or ionic cross-linking or polymerization reaction initiated by the photo initiator when the photo initiator is exposed to radiation from the optical fibre.

7. A stent system according to any preceding claim further comprising one or more bioactive components including one or more of a bioactive agent, a bioactive principle, a bioactive compound, a bioactive molecule, a bioactive drug, a therapeutic agent, a therapeutic principle, a therapeutic compound, a therapeutic molecule, and/or a therapeutic drug.

8. A stent system according to claim 7, wherein the one or more bioactive components is a synthetic or a natural compound of a molecular weight ranging from 100 to 500.000 Da, such as from 1.000 to 500.000 Da, or such as from 10.000 to 500.000 Da, or such as from 100 to 10.000 Da, or such as from 100 to 1.000 Da.

9. A stent system according to any of the claims 7-8, wherein the one or more bioactive components includes a bioactive component for one or more of the following:
• preventing that the immune response in the body is activated
• preventing that the tissue in the body cavity/vessel is injured due to the stent mesh
• preventing inflammation or similar inside the body
• postponing that the immune response in the body is activated
• postponing inflammation or similar inside the body.

10. A stent system according to any of the claims 7-9, wherein the one or more bioactive components includes a therapeutically active component for:
• postponing a disease, and inflammation or similar inside the body, and/or
• treating a disease, and inflammation or similar inside the body.

11. A stent system according to any of the claims 7-10, wherein the one or more bioactive components are comprised in colloidal formulations.

12. A stent system according to claim 11, wherein the colloidal formulations includes a colloidal drug delivery system (DDS) such as liposomes, and wherein one or more drug components in the DDS optionally is a hydrophobic or hydrophilic drug molecule.

13. A stent system according to any of the claims 7-12, wherein the one or more bioactive components, possibly comprised inside the colloidal formulation, are contained in the biocompatible solution or inside micro reservoirs in the stent mesh.

14. A stent system according to any preceding claim, wherein the stent mesh is made from a biodegradable material or a biocompatible and/or biodegradable metal.

15. A stent system for use in treating a disease and/or an inflammation inside the body.
